# EUROPÄISCHE PATENTSCHRIFT

(11) **EP 3 301 441 B1**
(45) Veröffentlichungstag und Bekanntmachung des Hinweises auf die Patenterteilung: **07.11.2018**
(21) Anmeldenummer: 16191950.1
(22) Anmeldetag: 30.09.2016
(51) Int. Cl.: G01N 33/00, G01N 25/18

(54) **WÄRMELEITFÄHIGKEITSDETEKTOR FÜR GASGEMISCHE MIT MINDESTENS DREI KOMPONENTEN**
THERMAL CONDUCTIVITY DETECTOR FOR GAS MIXTURES HAVING AT LEAST THREE COMPONENTS
DÉTECTEUR DE CONDUCTIVITÉ THERMIQUE POUR MIXTURES DE GAZ AYANT TROIS OU PLUS CONSTITUENTS

(43) Veröffentlichungstag der Anmeldung: 04.04.2018
(73) Patentinhaber: Siemens Aktiengesellschaft, 80333 München (DE)
(72) Erfinder: Ludwig, Michael, 76149 Karlsruhe (DE); Marcaux, Günter, 76187 Karlsruhe (DE)

(56) Entgegenhaltungen:
- EP-A1- 0 698 786
- EP-A2- 0 285 833
- DE-A1- 2 505 669
- US-A1- 2007 209 977
- US-A1- 2011 252 868

## Beschreibung

Die Erfindung betrifft einen Wärmeleitfähigkeitsdetektor.

Thermoelektrische Wärmeleitfähigkeitsdetektoren oder Strömungsfühler mit einem Heizelement und mindestens einem Thermoelement zur Temperaturmessung sind bekannt, so z. B. aus:
G. De Graaf et al.: "Surface-micromachined thermal conductivity detectors for gas sensing", Proc. of the IEEE International Instrumentation and Measurement Technology Conference (I2MTC), Graz, 13-16 May 2012, 1861-1864;
S. Udina et al.: "A micromachined thermoelectric sensor for natural gas analysis: Thermal model and experimental results", Sensors and Actuators B 134 (2008) 551-558;
DE 10 2005 033 867 A1;
US 2006/0220164 A1;
US 6,378,365 B1;
US 2007/241093 A1.

Aus der EP 0 187 723 A2 oder DE 199 09 469 C1 ist es ferner bekannt, das Thermoelement gleichzeitig aufzuheizen (Peltiereffekt) und mit ihm die Temperatur zu messen (Seebeckeffekt). Die Aufheizung erfolgt mit Wechselstrom.

Aus der US 2007/209977 A1 ist schließlich ein Strömungsmesser (Anemometer) bekannt, bei dem ein Thermoelement in einem Strömungspfad angeordnet ist und von einem Fluid, hier einer Flüssigkeit, umströmt wird. Das Thermoelement wird pulsförmig aufgeheizt, indem es über einen Schalter periodisch an eine Stromversorgungsquelle geschaltet wird. Zwischen den Heizimpulsen kühlt das Thermoelement ab, wobei die Zeitkonstante der Abkühlung von der Strömungsgeschwindigkeit des Fluids abhängig ist. In der Abkühlphase wird zu zwei unterschiedlichen Zeitpunkten die Temperatur des Thermoelements durch Messung seiner elektrischen Spannung gemessen. Aus den beiden erhaltenen Temperaturwerten wird die Strömungsgeschwindigkeit des Fluids berechnet. Der bekannte Strömungsmesser dient zur Bewertung der Leistungsfähigkeit von Umkehrosmose-Membranen und kann um eine Messzelle zur Messung der elektrischen Leitfähigkeit der Flüssigkeit ergänzt werden.
Wie z. B. in der oben genannten Veröffentlichung von S. Udina et al. angegeben, kann der thermoelektrische Wärmeleitfähigkeitsdetektor Bestandteil eines Gasanalysators zur Ermittlung des Verhältnisses der Komponenten eines binären Gasgemisches sein.

In einer weiteren Veröffentlichung von S. Udina et al: "A micromachined thermoelectric sensor for natural gas analysis: Multivariate calibration results", Sensors and Actuators B 166 (2011) 538-348 werden auch Gasgemische mit drei oder mehr Komponenten betrachtet.

Es ist darüber hinaus z. B. aus der EP 0 285 833 A2 bekannt, einen herkömmliche Wärmeleitfähigkeitsdetektor mit resistiven Elementen bei N-1 unterschiedlichen Temperaturen zu betreiben, um Gasgemische mit N-Komponenten zu analysieren. Dabei ist es aber erforderlich, die unterschiedlichen Heiztemperaturen sehr genau einzustellen. Ferner muss die thermische Zeitkonstante des Wärmeleitfähigkeitsdetektors genügend klein sein, um die unterschiedlichen Temperaturen schnell einstellen zu können.
Der Erfindung liegt die Aufgabe zugrunde, mit sehr einfachen Mitteln eine sehr schnelle und genau Analyse von ternären oder multiplen Gasgemischen zu ermöglichen.
Gemäß der Erfindung wird die Aufgabe durch den in Anspruch 1 angegebenen Gasanalysator gelöst.
Vorteilhafte Weiterbildungen des erfindungsgemäßen Gasanalysators sind den Unteransprüchen zu entnehmen.
Gegenstand der Erfindung ist somit ein Gasanalysator für ein Gasgemisch mit drei oder mehr Komponenten, mit
- einem das Gasgemisch führenden Kanal,
- einer Thermoelement-Sonde mit einer Messstelle im Inneren des Kanals und einer Vergleichsstelle in thermischem Kontakt mit der Wand des Kanals,
- einer Steuereinrichtung, die zur pulsförmigen Ansteuerung der Thermoelement-Sonde mit Stromimpulsen ausgebildet ist,
- einer Messeinrichtung, die zur Messung der Spannung an der Vergleichsstelle der Thermoelement-Sonde in den Pausen zwischen den Stromimpulsen ausgebildet ist, und
- einer Auswerteeinrichtung, die zur Ermittlung der Temperaturdifferenz zwischen der Messstelle und der Vergleichsstelle aus der gemessenen Spannung und zur Berechnung des Verhältnisses der Komponenten des Gasgemisches aus zu mindestens zwei unterschiedlichen Zeitpunkten in jeder Pause ermittelten Temperaturdifferenzwerten ausgebildet ist,
- wobei die Steuereinrichtung ferner dazu ausgebildet ist, Stromimpulse mit mindestens zwei unterschiedlichen Energieinhalten zu erzeugen, und
- wobei die Auswerteeinrichtung ferner dazu ausgebildet ist, entweder
   - die Zeitpunkte zur Ermittlung der Temperaturdifferenzwerte in vorgegebenen Abständen zu dem Ende des jeweils vorangehenden Stromimpulses festzulegen und somit in der Pause nach einem Stromimpuls mit größerem Energiegehalt zu dem ersten Zeitpunkt einen höheren Temperaturdifferenzwert zu erfassen und für die Berechnung zu verwenden, als nach einem Stromimpuls mit kleinerem Energiegehalt, oder
   - die Zeitpunkte zu ermitteln, zu denen die Temperaturdifferenz mindestens zwei unterschiedliche vorgegebene Temperaturdifferenzwerte erreicht, und die Abstände der ermittelten Zeitpunkte zu dem Ende des jeweils vorangehenden Stromimpulses zur Berechnung des Verhältnisses der Komponenten des Gasgemisches heranzuziehen.

Die Messstelle, d. h. die heiße Verbindungsstelle der Thermoelement-Sonde wird also im Verlauf der pulsförmigen Ansteuerung unterschiedlich stark aufgeheizt, so dass zumindest in den Anfangsphasen der auf die unterschiedlichen Stromimpulse folgenden Pausen unterschiedlich hohe Temperaturdifferenzen zwischen der heißen Messstelle der Thermoelement-Sonde und der vergleichsweise kalten Wand des das Gasgemisch führenden Kanals vorliegen, die mittels Thermoelement-Sonde gemessen werden. Dabei kommt es in vorteilhafter Weise überhaupt nicht auf die Genauigkeit der Heiztemperatur der Thermoelement-Sonde an, weil die Temperaturdifferenz bzw. ihr Verlauf erst nach der Aufheizung der Thermoelement-Sonde in den Pausen zwischen den Stromimpulsen gemessen wird. Entscheidend ist lediglich, dass der in der Pause nach einem Stromimpuls mit größerem Energiegehalt zuerst erfasste Temperaturdifferenzwert höher ist, als der bei einem Stromimpuls mit kleinerem Energiegehalt zuerst erfasste Temperaturdifferenzwert, wenn die Zeitpunkte zur Ermittlung der Temperaturdifferenzwerte in vorgegebenen zeitlichen Abständen zu dem Ende des jeweils vorangehenden Stromimpulses festgelegt sind, weil bei einer stärkeren Aufheizung der Thermoelement-Sonde nach Ablauf einer auf die Aufheizung folgenden vorgegebenen Zeit eine höhere Temperatur vorliegt, als dies bei einer schwächeren Aufheizung nach derselben Zeit der Fall ist.

Da zum einen die Temperaturdifferenz zwischen der Messstelle der Thermoelement-Sonde und der Wand des das Gasgemisch führenden Kanals nach jedem Stromimpuls in Abhängigkeit von der Wärmeleitfähigkeit des Gasgemischs exponentiell abklingt und zum anderen die Wärmeleitfähigkeit von Gasen im allgemeinen eine Funktion der Gastemperatur und diese Funktion für verschiedenen Gase unterschiedlich ist, können anhand von mindestens zwei unterschiedlichen Verläufen der in den Pausen zwischen den Stromimpulsen abklingenden Temperaturdifferenz mindestens drei unterschiedliche Komponenten in dem Gasgemisch im Verhältnis zueinander quantitativ bestimmt werden. Dabei werden die mindestens zwei unterschiedlichen Verläufe der abklingenden Temperaturdifferenz durch unterschiedlich starkes Aufheizen der Messstelle mit Stromimpulsen unterschiedlichen Energieinhalts erzeugt.

Um den exponentiell abklingenden Verlauf der Temperaturdifferenz zu erhalten, reicht es aus, in jeder Pause mindestens zwei Temperaturdifferenzwerte zu mindestens zwei unterschiedlichen Zeitpunkten zu ermitteln. Wie oben bereits erwähnt, können diese Zeitpunkte in vorgegebenen Abständen zu dem Ende des jeweils vorangehenden Stromimpulses festgelegt sein.

Alternativ können mindestens zwei unterschiedliche Temperaturdifferenzwerte vorgegeben werden und in dem exponentiell abklingenden Verlauf der Temperaturdifferenz die Zeitpunkte ermittelt werden, zu denen diese vorgegebenen Temperaturdifferenzwerte erreicht werden. Die Abstände der ermittelten Zeitpunkte zu dem Ende des jeweils vorangehenden Stromimpulses werden dann zur Berechnung des Verhältnisses der Komponenten des Gasgemisches herangezogen.

Da es, wie oben bereits erwähnt, nicht auf die Genauigkeit der jeweiligen Heiztemperatur der Thermoelement-Sonde ankommt, spielt es auch keine Rolle, wie die Stromimpulse erzeugt werden. So können die Stromimpulse mit unterschiedlichen Energieinhalten unterschiedliche Impulshöhen und/oder unterschiedliche Impulsbreiten aufweisen. Wird z. B. der Energieinhalt der Stromimpulse ausschließlich über deren Breite bzw. Dauer eingestellt, so braucht die Steuereinrichtung, welche die Stromimpulse erzeugt, keine unterschiedlichen elektrischen Spannungen bereitzustellen.

Zur weiteren Erläuterung der Erfindung wird im Folgenden auf die Figuren der Zeichnung Bezug genommen; im Einzelnen zeigen:
- FIG. 1: ein schematisches Ausführungsbeispiel des erfindungsgemäßen Gasanalysators,
- FIG. 2: ein erstes Ausführungsbeispiel für zwei Stromimpulse mit unterschiedlichem Energieinhalt und
- FIG. 3: ein weiteres Ausführungsbeispiel für die beiden Stromimpulse.

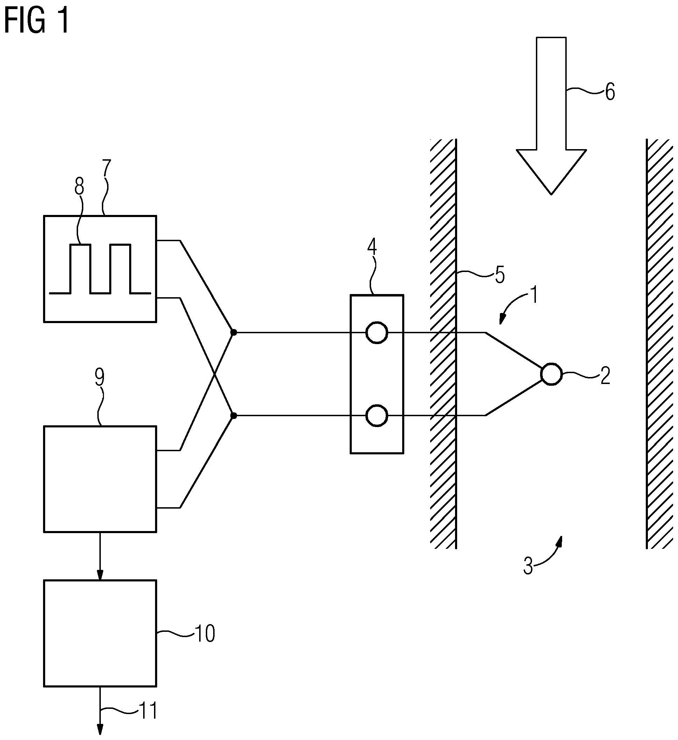

FIG. 1 zeigt in schematischer Darstellung ein Blockschaltbild eines Gasanalysators mit einer Thermoelement-Sonde 1, die mit ihrer Messstelle 2, also der heißen Verbindungsstelle, im Inneren des Kanals 3 und mit ihrer Vergleichsstelle 4, also der kalten Verbindungsstelle im Bereich der der Wand 5 des Kanals 3 in thermischem Kontakt mit dieser angeordnet ist. Durch den Kanal 43 fließt ein ternäres oder multiples Gasgemisch 6, dessen Zusammensetzung quantitativ zu bestimmen ist. Bei der Thermoelement-Sonde 1 kann es sich um ein einzelnes Thermoelement oder um eine Kette von Thermoelementen handeln.

Die Thermoelement-Sonde 1 ist mit ihrer kalten Verbindungsstelle 4 an einer Steuereinrichtung 7 angeschlossen, welche die Thermoelement-Sonde 1 mit einer Folge von Stromimpulsen 8 an steuert. Weiterhin ist die Thermoelement-Sonde 1 an einer Messeinrichtung 9 angeschlossen, die in den Pausen zwischen den Stromimpulsen 8 an der Vergleichsstelle 4 die von der Thermoelement-Sonde 1 erzeugte elektrische Spannung misst. Der Messeinrichtung 9 ist eine Auswerteeinrichtung 10 nachgeordnet, die aus der in dem Pausen zwischen den Stromimpulsen 8 gemessenen Spannung die Temperaturdifferenz zwischen der Messstelle 2 und der Wand 5 des Kanals 3 misst und daraus und bekannten Parametern wie insbesondere den spezifischen Wärmeleitfähigkeiten der zu messenden Komponenten des Gasgemischs 3 das Verhältnis dieser Komponenten berechnet und als Messwert 11 ausgibt.

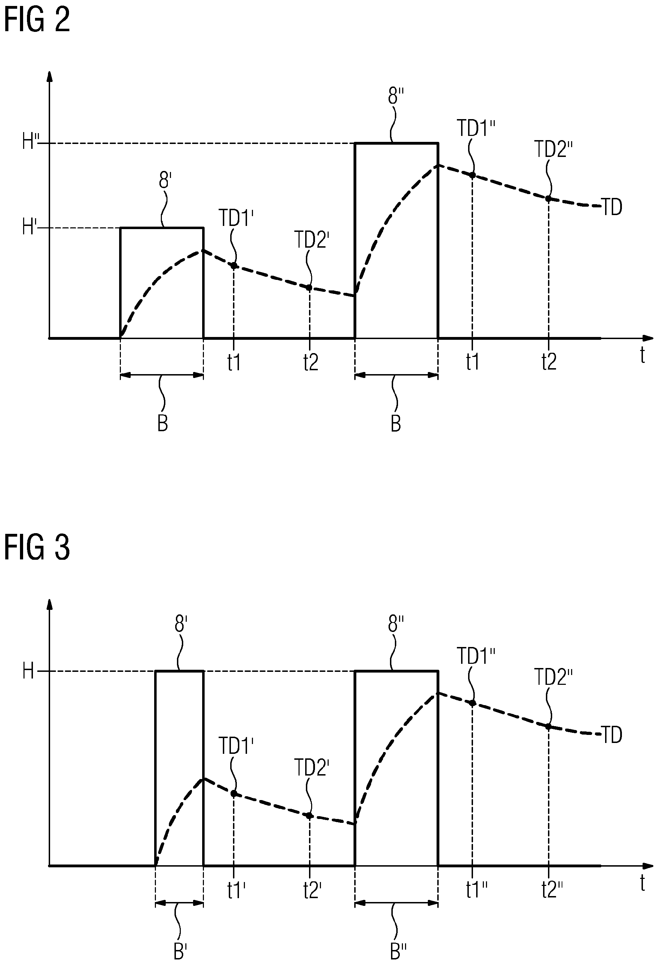

FIG. 2 zeigt ein Beispiel für zwei von der Steuereinrichtung 7 erzeugte Stromimpulse 81 und 82 mit jeweils unterschiedlichem Energieinhalt. Bei dem gezeigten Beispiel weisen die beiden Stromimpulse 8' und 8" bei gleicher Breite (Dauer) B unterschiedliche Impulshöhen (H', H") auf. Ferner ist der Verlauf der Temperaturdifferenz TD zwischen der Messstelle 2 und der Wand 5 des Kanals 3 bzw. der Vergleichsstelle 4 dargestellt. In jeder Pause zwischen zwei Stromimpulsen 8 werden zu zwei unterschiedlichen Zeitpunkten t1 und t2 Temperaturdifferenzwerte TD1', TD2' und TD1", TD2" erfasst. Die Zeitpunkte t1, t2 liegen jeweils in vorgegebenen Abständen zu dem Ende des jeweils vorangehenden Stromimpulses 8' bzw. 8". Der nach dem energiereicheren Stromimpuls 8' zuerst erfasste Temperaturdifferenzwert TD1' ist höher, als der nach dem energieärmeren Stromimpuls 8" zuerst erfasste Temperaturdifferenzwert TD1".

Wie FIG. 2 zeigt, können anstelle der zeitlichen Abstände der Zeitpunkte t1, t2 zu den jeweils vorangehenden Stromimpulsen 8' bzw. 8' die Temperaturdifferenzwerte TD1', TD2', TD1", TD2" vorgegeben werden, wobei dann die Zeitpunkte t1', t2' und t1", t2" erfasst werden, bei die vorgegebenen Temperaturdifferenzwerte TD1', TD2' bzw. TD1", TD2' erreicht werden. Weiterhin zeigt FIG. 2 eine alternatives Beispiel für die beiden Stromimpulse 8' und 8", die hier bei gleicher Impulshöhe H unterschiedliche Breiten B', B" aufweisen.

## Patentansprüche

1. Gasanalysator für ein Gasgemisch (6) mit drei oder mehr Komponenten, mit
- einem das Gasgemisch (6) führenden Kanal (3),
- einer Thermoelement-Sonde (1) mit einer Messstelle (2) im Inneren des Kanals (3) und einer Vergleichsstelle (4) in thermischem Kontakt mit der Wand des Kanals (3),
- einer Steuereinrichtung (7), die zur pulsförmigen Ansteuerung der Thermoelement-Sonde (1) mit Stromimpulsen (8, 8', 8") ausgebildet ist,
- einer Messeinrichtung (9), die zur Messung der Spannung an der Vergleichsstelle (4) der Thermoelement-Sonde (1) in den Pausen zwischen den Stromimpulsen (8, 8', 8") ausgebildet ist, und
- einer Auswerteeinrichtung (10), die zur Ermittlung der Temperaturdifferenz (TD) zwischen der Messstelle (2) und der Vergleichsstelle (4) aus der gemessenen Spannung und zur Berechnung des Verhältnisses der Komponenten des Gasgemischs (6) aus zu mindestens zwei unterschiedlichen Zeitpunkten (t1, t2) in jeder Pause ermittelten Temperaturdifferenzwerten (TD1', TD2', TD1", TD2") ausgebildet ist,
**dadurch gekennzeichnet**, - dass die Steuereinrichtung (7) ferner dazu ausgebildet ist, Stromimpulse (8', 8") mit mindestens zwei unterschiedlichen Energieinhalten zu erzeugen, und
- dass die Auswerteeinrichtung (10) ferner dazu ausgebildet ist, entweder
- die Zeitpunkte (t1, t2) zur Ermittlung der Temperaturdifferenzwerte (TD1', TD2', TD1", TD2") in vorgegebenen Abständen zu dem Ende des jeweils vorangehenden Stromimpulses (8', 8") festzulegen und somit in der Pause nach einem Stromimpuls (8") mit größerem Energiegehalt zu dem ersten Zeitpunkt (t1) einen höheren Temperaturdifferenzwert (TD1") zu erfassen und für die Berechnung zu verwenden, als nach einem Stromimpuls (8') mit kleinerem Energiegehalt, oder
- die Zeitpunkte zu ermitteln, zu denen die Temperaturdifferenz (TD) mindestens zwei unterschiedliche vorgegebene Temperaturdifferenzwerte (TD1', TD2', TD1", TD2") erreicht, und die Abstände der ermittelten Zeitpunkte (t1', t2', t1", t2") zu dem Ende des jeweils vorangehenden Stromimpulses (8', 8") zur Berechnung des Verhältnisses der Komponenten des Gasgemisches (6) heranzuziehen.

2. Gasanalysator nach Anspruch 1, **dadurch gekennzeichnet, dass** die Steuereinrichtung (7) dazu ausgebildet ist, die Stromimpulse (8, 8', 8") mit unterschiedlichen Energieinhalten durch unterschiedliche Impulshöhen (H', H") zu erzeugen.

3. Gasanalysator nach Anspruch 1 oder 2, **dadurch gekennzeichnet, dass** die Steuereinrichtung (7) dazu ausgebildet ist, die Stromimpulse (8, 8', 8") mit unterschiedlichen Energieinhalten durch unterschiedliche Impulsbreiten (B', B") zu erzeugen.

## Claims

1. Gas analyser for a gas mixture (6) with three or more components, having
- a channel (3) guiding the gas mixture (6),
- a thermal element probe (1) with a measuring point (2) in the interior of the channel (3) and a reference junction (4) in thermal contact with the wall of the channel (3),
- a control facility (7), which is embodied for the pulse-shaped activation of the thermal element probe (1) with current pulses (8, 8', 8"),
- a measuring facility (9), which is embodied to measure the voltage at the reference junction (4) of the thermal element probe (1) in the pauses between the current pulses (8, 8', 8"), and
- an evaluation facility (10), which is embodied to determine the temperature difference (TD) between the measuring point (2) and the reference junction (4) from the measured voltage and to calculate the ratio of the components in the gas mixture (6) from temperature difference values (TD1', TD2', TD1", TD2") determined at at least two different points in time (t1, t2) in each pause, **characterised in that** the control facility (7) is further embodied to generate current pulses (8', 8") with at least two different energy contents, and
that the evaluation facility (10) is further embodied, either
- to define the points in time (t1, t2) for determining the temperature difference values (TD1', TD2' TD1", TD2") at predetermined intervals at the end of the current pulse (8', 8") which precedes in each instance and thus in the pause after a current pulse (8") with a larger energy content at the first point in time (t1) to detect a higher temperature difference value (TD1"), and use same for the calculation, than after a current pulse (8') with a smaller energy content, or
- to determine the points in time at which the temperature difference (TD) reaches at least two different predetermined temperature difference values (TD1', TD2', TD1", TD2") and to use the distances between the determined points in time (t1', t2', t1", t2") at the end of the respectively preceding current pulse (8', 8") in order to calculate the ratio of the components in the gas mixture (6).

2. Gas analyser according to claim 1, **characterised in that** the control facility (7) is embodied to generate the current pulses (8, 8', 8") with different energy contents by means of different pulse heights (H', H").

3. Gas analyser according to claim 1 or 2, **characterised in that** the control facility (7) is embodied to generate the current pulses (8, 8', 8") with different energy contents by means of different pulse widths (B', B").

## Revendications

1. Analyseur de gaz pour un mélange (6) gazeux ayant trois ou plus de trois constituants, comprenant
- un conduit (3), conduisant le mélange (6) gazeux,
- une sonde (1) à thermocouple, ayant un point (2) de mesure à l'intérieur du conduit (3) et un point (4) de comparaison en contact thermique avec la paroi du conduit (3),
- un dispositif (7) de commande, constitué pour l'excitation pulsée de la sonde (1) à thermocouple par des impulsions (8,8', 8") de courant,
- un dispositif (9) de mesure, constitué pour mesurer la tension au point (4) de comparaison de la sonde (1) à thermocouple dans les intervalles entre les impulsions (8, 8', 8") de courant et
- un dispositif (10) d'exploitation, constitué pour déterminer la différence (TD) de température entre le point (2) de mesure et le point (4) de comparaison, à partir de la tension mesurée, et pour calculer le rapport des constituants du mélange (6) gazeux, à partir de valeurs (TD1', TD2', TD1", TD2") de différence de température déterminées dans chaque intervalle à au moins deux instants (t1, t2) différents,
**caractérisé en ce que** le dispositif (7) de commande est constitué, en outre, pour produire des impulsions (8', 8") de courant ayant au moins deux contenus énergétiques différents et
- **en ce que** le dispositif (10) d'exploitation est constitué, en outre, pour, ou bien
- fixer les instants (t1, t2) de détermination des valeurs (TD1', TD2', TD1", TD2") de différence de température à des distances données à l'avance de la fin de l'impulsion (8', 8") de courant précédente respective et à détecter ainsi, dans l'intervalle, après une impulsion (8") de courant ayant un contenu énergétique plus grand par rapport au premier instant (t1), une valeur (TD1") de différence de température plus grande et à l'utiliser pour le calcul plutôt qu'après une impulsion (8') de courant ayant un contenu énergétique plus petit, ou bien
- pour déterminer les instants où la différence (TD) de température atteint au moins deux valeurs (TD1', TD2', TD1", TD2") de différence de température différentes données à l'avance et pour tirer parti des distances des instants (t1', t2', t1", t2") déterminés par rapport à la fin de l'impulsion (8', 8") de courant précédente respective pour calculer le rapport des constituants du mélange (6) gazeux.

2. Analyseur de gaz suivant la revendication 1, **caractérisé en ce que** le dispositif (7) de commande est constitué pour produire les impulsions (8, 8', 8") de courant ayant des contenus énergétiques différents par des amplitudes (H', H") d'impulsion différentes.

3. Analyseur de gaz suivant la revendication 1 ou 2, **caractérisé en ce que** le dispositif (7) de commande est constitué pour produire les impulsions (8, 8', 8") de courant ayant des contenus énergétiques différents par des largeurs (B', B") d'impulsion différentes.
